# FASCICULE DE BREVET EUROPEEN

(11) **EP 3 629 939 B1**
(45) Date de publication et mention de la délivrance du brevet: **23.09.2020**
(21) Numéro de dépôt: 17822010.9
(22) Date de dépôt: 01.11.2017
(51) Int. Cl.: A61B 10/00, G01N 33/52, A61F 13/70, A61F 13/505, A61F 13/49, A61F 13/84

(54) **KIT ET MÉTHODE POUR PRÉLÈVEMENT D'URINE**
KIT UND VERFAHREN ZUR SAMMLUNG VON URIN
KIT AND METHOD FOR THE COLLECTION OF URINE

(30) Priorité: 24.05.2017 CH 6852017
(43) Date de publication de la demande: 08.04.2020
(73) Titulaire: PALMA GROUP SA, 1700 Fribourg (CH)
(72) Inventeur: PORVAN, Pavlo, 1700 Fribourg (CH)
(74) Mandataire: Bugnion Genève
(86) Numéro de dépôt international: PCT/IB2017/056789
(87) Numéro de publication internationale: WO 2018/215822

(56) Documents cités:
- US-A- 3 918 433
- US-A- 5 595 187
- US-A1- 2004 082 878
- US-A1- 2007 185 466
- US-A1- 2008 274 014
- US-A1- 2015 094 227
- US-A1- 2015 152 468
- US-A1- 2016 029 957

## Description

### Domaine technique

L'invention se rapporte à une méthode d'identification qualitative de composés dans l'urine humaine. Elle permet notamment d'identifier la présence de composés normalement absents, tels que glucose, cétones, albumine, leucocytes, monocytes ou érythrocytes. La présente invention permet en outre l'identification d'écarts de la norme de certains indices de l'urine comme, par exemple, le pH.
La présente invention couvre également le kit d'éléments nécessaires à la mise en œuvre de la méthode.

L'invention peut être utilisée à des fins de recherches dans le domaine de la médecine, ainsi que pour diagnostiquer l'état de santé des êtres humains. Le diagnostic se base alors sur l'analyse d'écarts de la norme des indices de l'urine, ou l'identification de un ou plusieurs composés.

L'invention peut être utilisée aussi bien dans des établissements médicaux que par des non-professionnels dans des conditions domestiques.

### Etat de la technique

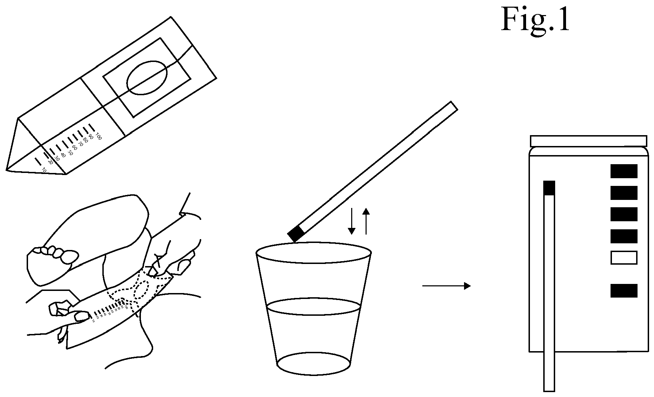

Déceler une pathologie à un stade précoce facilite significativement son traitement, chez les adultes et chez les enfants, pour qui les maladies peuvent se développer rapidement. En particulier, la première année de vie d'un enfant peut être cruciale dans l'évolution de sa santé. L'élévation constante du niveau des affections et de la morbidité infantiles préoccupe de nombreux gouvernements et a conduit le Canada à initier un programme gouvernemental d'analyse de l'urine et du sang des nouveau-nés.
L'analyse d'urine permet de diagnostiquer différents dérèglements ou d'identifier différentes pathologies. Cela permet d'initier rapidement la prophylaxie et/ou le traitement approprié. Les indications thérapeutiques ainsi visées comportent les perturbations du métabolisme, l'obésité, le diabète sucré, l'infection au rotavirus, les maladies génétiques, les perturbations des fonctions de la glande thyroïdienne. Pour les enfants portant encore des couches, et plus particulièrement pour les enfants âgés de 0 à 18 mois, il peut être difficile de recueillir l'urine à des fins d'analyse. Indépendamment de l'âge, le problème se pose aussi pour les personnes dont les possibilités physiques sont limitées. En particulier, il peut être difficile de recueillir un échantillon d'urine de personnes âgées, handicapées, alitées, ou ayant une mobilité réduite. En effet, les méthodes connues nécessitent de recueillir l'échantillon d'urine dans un récipient avant de procéder à l'analyse. En pratique, l'analyse de l'échantillon est effectuée par l'immersion de bandelettes urinaires indicatrices dans l'urine récoltée. Les bandelettes urinaires indicatrices existantes se présentent sous la forme de bandes en matière plastique sur lesquelles sont disposés des réactifs. Elles sont généralement conditionnées dans des tubes, comprenant chacun de 50 à 100 bandelettes. Les tubes de bandelettes urinaires comportent un absorbeur d'humidité. Un tel conditionnement, s'il est adapté pour les analyses fréquentes pratiquées dans les laboratoires ou pour les malades chroniques nécessitant un suivi régulier, se révèle peu pratique pour une utilisation à domicile. Les opérations faites à domiciles se limitent d'ailleurs souvent à la collecte de l'échantillon d'urine dans un récipient, qu'il faut ensuite apporter à un laboratoire d'analyse.
Le volume d'urine à récolter doit être suffisant pour permettre l'immersion de tous les réactifs de la bandelette urinaire. L'évaluation des résultats de l'analyse de l'urine est basée sur le changement de la couleur d'un ou plusieurs réactifs indicateurs sous l'action des composés de l'urine. Les couleurs des réactifs sont alors comparées à une échelle colorimétrique. Les réactifs disposés sur la bandelette urinaire indicatrice sont chacun spécifiques du composé de l'urine dont on cherche la présence. La méthode usuelle d'immersion/absorption nécessite une manipulation du patient pour la collecte de l'échantillon à analyser. Cette manipulation peut être malaisée et parfois contraignante lors d'analyses répétées pour un suivi journalier. La méthode usuelle d'immersion/absorption nécessite en outre de retirer la bandelette urinaire de l'échantillon suffisamment rapidement pour éviter que les réactifs se dégradent ou se dispersent dans l'échantillon. Par ailleurs le retrait de la bandelette urinaire du récipient contenant l'échantillon conduit inévitablement à l'écoulement de l'urine le long de la bandelette urinaire, ce qui peut conduire à l'entrainement et au mélange des réactifs disposés sur la bandelette urinaire. Il est donc nécessaire de disposer rapidement la bandelette à l'horizontal pour éviter l'écoulement de l'urine sur les réactifs. Le fait de poser la bandelette imbibée de l'urine du patient sur un support horizontal peut aisément contrevenir aux règles d'hygiène.
La méthode usuelle d'immersion/absorption est illustrée par la figure 1.

On connaît par ailleurs le document US 2008/274014 A1 qui décrit une méthode d'identification d'écarts par rapport à une norme d'un ou plusieurs composés de l'urine et de paramètres physico-chimiques. On connaît également le document US 2007/185466 A1, qui divulgue quant à lui l'utilisation d'une couche diagnostique.

### Exposé de l'invention

La présente invention permet de pallier aux inconvénients précités.

En particulier, la méthode mise en œuvre dans la présente invention permet de pratiquer l'analyse d'urine par simple accumulation de l'urine dans un élément d'accumulation, qui est ensuite utilisé pour humecter une bandelette urinaire indicatrice.
L'élément d'accumulation est de préférence disposé de façon amovible sur une couche, de telle manière qu'il n'est pas nécessaire de manipuler le patient pour récolter un échantillon d'urine. La méthode d'humectation/accumulation comprend notamment une étape de détachement de l'élément d'accumulation, une étape d'humectation d'une bandelette urinaire indicatrice par l'élément d'accumulation détaché de la couche, et une étape d'observation des couleurs de la bandelette urinaire indicatrice, et de comparaison avec une échelle colorimétrique.

L'élément d'accumulation d'urine peut comprendre un moyen de fixation amovible et un dispositif d'absorption. Le détachement de l'élément d'accumulation de l'urine inclut alors le décollement du moyen de fixation amovible, ce qui permet de prélever le dispositif d'absorption imbibé de l'urine à analyser.
Le moyen de fixation amovible est disposé dans une zone centrale et extérieure de la couche, permettant ainsi d'accéder à un échantillon d'urine sans devoir ôter la couche.

La bandelette urinaire indicatrice est de préférence associée à, ou disposée sur, un dispositif d'analyse, comprenant une zone d'essai, une zone comportant une ou plusieurs échelles colorimétriques, une zone de disposition de l'accumulateur d'urine, une zone de disposition d'un absorbeur d'humidité et une zone d'extrusion en relief.

L'humectation de la bandelette urinaire indicatrice par l'élément d'accumulation de l'urine est maintenue pour une durée de 10 à 120 secondes.

La lecture des résultats peut optionnellement être effectuée après une temporisation de comprise entre 0 et environ 120 secondes.

La lecture du résultat peut inclure la comparaison de la couleur des réactifs indicateurs avec la ou les échelles colorimétriques correspondantes. Alternativement ou en plus, la lecture du résultat peut inclure la reconnaissance de la couleur des réactifs indicateurs par un dispositif électronique équipé d'une interface.

La présente invention comprend en outre une couche diagnostique comprenant au moins un accumulateur d'urine, disposé à l'extérieur de la couche et en position centrale, lequel est fixé de manière amovible à la couche par un moyen de fixation amovible.
La présente invention comprend en outre un dispositif d'analyse comprenant au moins une zone d'essai, une zone comportant une ou plusieurs échelles colorimétriques, et une zone de disposition d'un l'accumulateur d'urine. Une zone supplémentaire pour la disposition d'un absorbeur d'humidité et/ou une zone d'extrusion en relief peuvent être prévues sur le dispositif d'analyse. Le dispositif d'analyse comporte de préférence une zone dédiée à la disposition d'un absorbeur d'humidité.

La présente invention comprend en outre un kit d'analyse comprenant au moins la couche et le dispositif d'analyse tel que décrit ci-dessus. Le kit d'analyse contient avantageusement une ou plusieurs bandelettes urinaires indicatrices, qui peuvent être libres ou bien fixées au dispositif d'analyse. Le dispositif d'analyse est de préférence conditionné dans un emballage hermétique. Il peut y être conditionné de manière individuelle. Le kit selon l'invention peut en outre comprendre une notice explicative ou une référence à un programme téléchargeable sur un dispositif électronique, ou une notice explicative et une référence à un programme téléchargeable sur un dispositif électronique.

### Brève description des figures

**Figure 1** : Représentation schématique de la méthode traditionnelle de prélèvement de l'urine et de son analyse chez un enfant âgé de 0 à 18 mois.
**Figures 2a****,** **2b****,** **2c****,** **2d** : Représentations de la couche avec l'accumulateur d'urine, lors des étapes de prélèvement d'urine, selon la présente invention.
**Figure 3** **:** Représentation schématique du dispositif d'analyse selon la présente invention.
**Figure 4** : Représentation schématique de la mise en œuvre de la méthode d'analyse selon la présente invention.

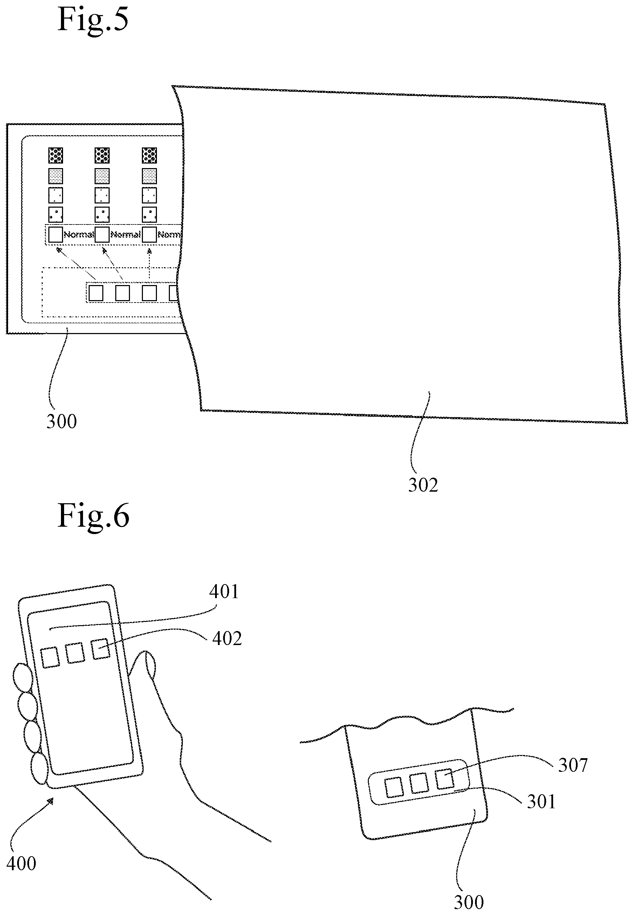

**Figure 5** **:** Représentation schématique du dispositif d'analyse et de son emballage hermétique après ouverture.
**Figure 6** : Représentation schématique de l'interface d'un dispositif électronique pour la conduite de l'analyse.

### Description détaillée de l'invention

La présente méthode d'analyse de l'urine permet d'identifier les écarts éventuels par rapport à la norme d'un ou plusieurs de ses composés ou paramètres, grâce au principe de l'HUMECTATION/ACCUMULATION. L'analyse est de préférence qualitative. L'écart par rapport à la norme de l'un ou plusieurs des composés de l'urine désigne la présence de composés normalement absent de l'urine, ou leur présence en quantité différente par rapport à un seuil prédéterminé. Une quantité supérieure à la norme d'un ou plusieurs composés de l'urine est préférentiellement détectée, mais une quantité insuffisante par rapport à la norme peut également être détectée.
Les composés de l'urine désignent l'ensemble des éléments chimiques et biologiques présents dans l'urine au moment du test, ce qui comprend les produits excédentaires non métabolisés et les produits de dégradation métabolique.
Tout au long de cette description et sauf mention contraire, la norme s'entend comme une valeur de référence correspondant à la valeur usuelle observée dans la population, une telle valeur de référence traduisant un état de santé convenable de la personne à l'origine de l'échantillon. L'écart à la norme désigne en conséquence une différence par rapport à cette norme pour le composé ou le paramètre mesuré, et peut être révélateur d'un disfonctionnement ou d'une pathologie. Les composés de l'urine incluent les sucres, et en particulier le glucose et ses dérivés, l'albumine, les peptides et protéines ainsi que les acides aminés, des dérivés azotés tels que l'urée, des cétones, des cellules et résidus de cellules tels que leucocytes et hématies.
D'autres paramètres que les composés de l'urine peuvent être déterminés lors de la présente méthode. En particulier, le pH peut être évalué pour en déterminer l'écart éventuel par rapport à la norme.
La détermination d'un écart ou de l'absence d'écart par rapport à la norme implique la réaction d'un indicateur coloré, de préférence un produit chimique spécifique du composé de l'urine à analyser. Il est entendu que tout composé de l'urine ou paramètre physico-chimique susceptible d'être décelé par réaction avec un indicateur coloré peut faire l'objet d'une analyse selon la présente invention.

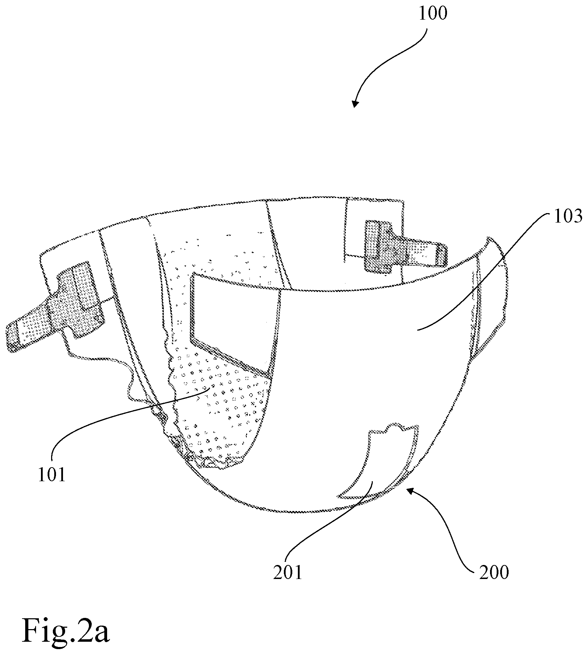

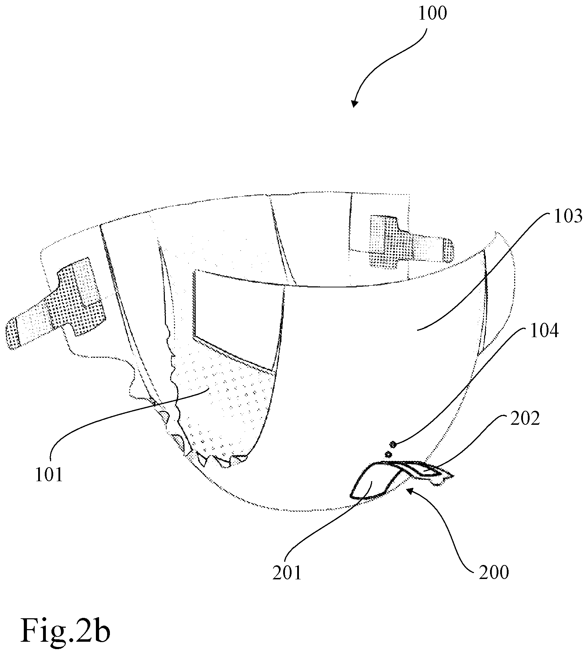

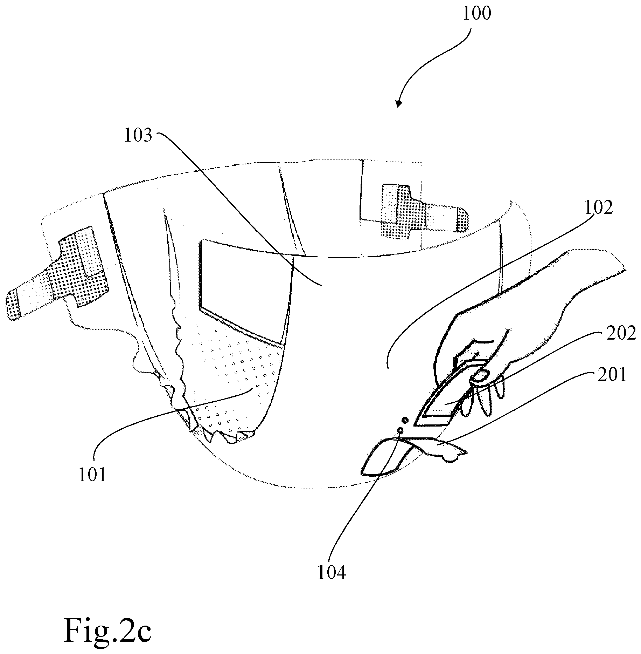

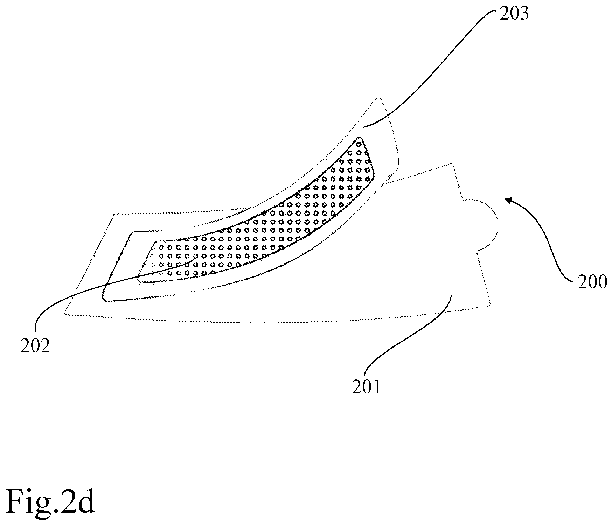

La présente méthode implique l'utilisation d'une couche **100,** comprenant un accumulateur d'urine **200.** La couche **100** est ainsi adaptée au prélèvement d'échantillons biologiques, tels que l'urine. Une telle couche peut être celle décrite en détails dans la demande de brevet d'invention Nº CH 00429/17 déposée le 30.03.2017 et représentée Sur les figures 2a, 2b et 2c. L'accumulateur d'urine **200** permet de récolter suffisamment d'urine pour faire réagir les réactifs indicateurs **307** par humectation d'une bandelette sur laquelle un ou plusieurs réactifs indicateurs **307** sont disposés. Une quantité de l'ordre de 2 à 3 ml d'urine peut ainsi être collectée grâce à l'accumulateur d'urine **200.** Une quantité de l'ordre de 3 ml d'urine peut ainsi être collectée dans certains modèles de couches grâce à l'accumulateur d'urine **200.**

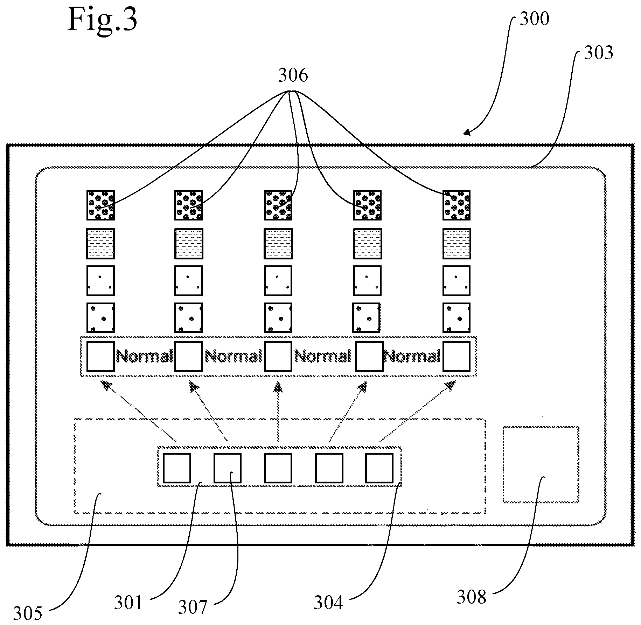

La présente méthode nécessite en outre l'utilisation d'un dispositif **300** d'analyse où est disposée une bandelette urinaire indicatrice **301,** dont un exemple est représenté dans la figure 3. Le dispositif **300** peut par exemple être sous la forme d'une plaque ou planchette.
Le dispositif d'analyse **300** est de préférence conditionné dans un emballage hermétique **302.** L'emballage hermétique **302** comporte avantageusement un seul dispositif d'analyse **300,** rendant ainsi le conditionnement individuel. Il n'est cependant pas exclus que plusieurs dispositifs d'analyse 300 soient inclus dans l'emballage hermétique **302** en fonction des utilisations envisagées. Le dispositif d'analyse **300** peut être fait en papier laminé et comporter sur les bords une extrusion en relief **303** pour empêcher le débordement de l'urine. Ce dispositif **300** possède notamment les zones suivantes:
- Zone d'essai **304,** où est disposée une bandelette urinaire **301** comportant les réactifs indicateurs **307** (zone d'essai)
- Zone de disposition **305** de l'accumulateur d'urine **200**
- Zone de l'échelle colorimétrique **306**
- Zone de disposition **308** de l'absorbeur d'humidité **3**
- Zone de l'extrusion en relief **303**

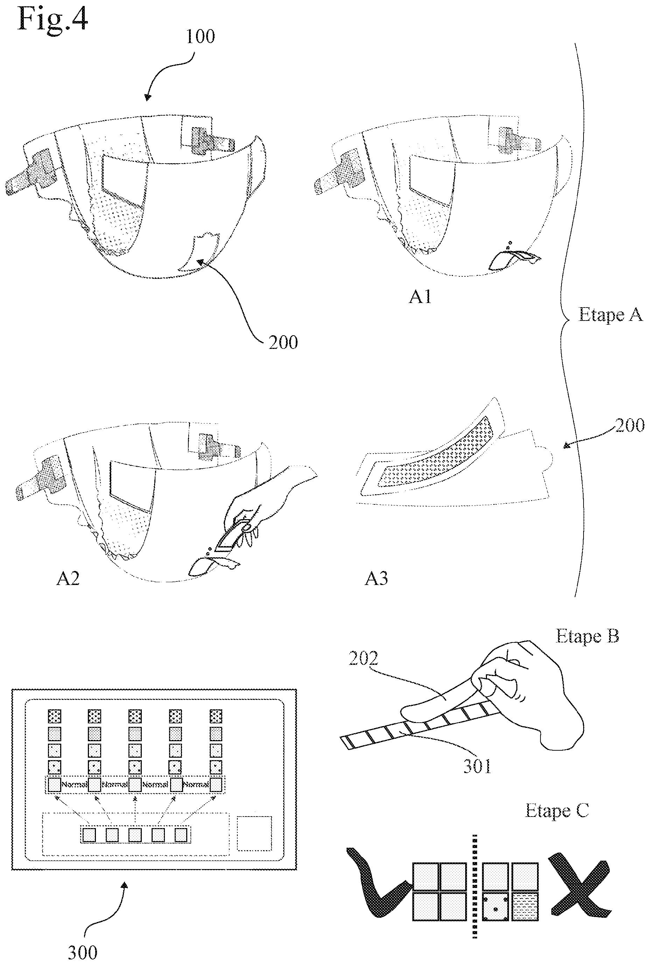

Les principales étapes de la méthode d'analyse sont illustrées par la figure 4. La méthode d'analyse comporte notamment une étape **A** de séparation de l'élément d'accumulation **200** de la couche diagnostique **100,** une étape **B** d'humectation d'une bandelette urinaire **301** par l'élément d'accumulation, et une étape **C** de lecture du résultat de l'analyse sur la bandelette urinaire **301.**
L'étape **A** peut comprendre une ou plusieurs des étapes intermédiaires **A1** de séparation d'un l'élément de fixation amovible **201, A2** de prélèvement d'un dispositif d'absorption **202** solidaire du moyen de fixation amovible **201,** et **A3** de séparation du dispositif d'absorption **202** d'une interface de fixation **203.** Lors de l'étape intermédiaire **A1,** l'élément de fixation amovible **201** peut être retiré totalement de la couche **100,** ou bien seulement partiellement. Il est à noter que dans le cas où le dispositif d'absorption **202** peut être facilement désolidarisé du moyen de fixation amovible **201,** ce dernier peut être retiré de la couche **100** de façon partielle, et permettre ainsi le prélèvement du dispositif d'absorption **202** lors de l'étape intermédiaire **A2.** Alternativement, le moyen de fixation amovible **201** peut être totalement retiré de la couche **100** avec le dispositif d'absorption **202.** Dans ces conditions, les étapes intermédiaires **A2** et **A3** peuvent être optionnelles, dans la mesure où le dispositif d'absorption **202** peut être directement utilisé pour l'humectation des réactifs indicateurs **307,** bien qu'étant encore lié au moyen de fixation amovible **201.** Dans le cas où le dispositif d'absorption **202** comporte une interface de fixation **203** qui le maintien associé au moyen de fixation amovible **201,** une étape intermédiaire **A3** peut être prévue pour séparer l'interface de fixation **203.** L'étape **A** décrite ci-dessus, ainsi que les étapes intermédiaires **A1, A2** et **A3** peuvent s'effectuer alors que la couche **100** est encore en place sur la personne, ce qui permet de limiter sa manipulation.

L'étape **B** d'humectation de bandelette **301** comprend notamment les étapes intermédiaires **B1,** d'application du dispositif d'absorption **202** sur la bandelette **301** comprenant un ou plusieurs réactifs indicateurs **307,** et **B2** de retrait du dispositif d'absorption **202.** Comme indiqué ci-dessus, le dispositif d'absorption **202** peut être appliqué seul ou combiné avec un ou plusieurs des autres constituants de l'accumulateur d'urine **200,** tels que le moyen de fixation amovible **201** et l'interface de fixation **203.** L'étape d'humectation s'effectue de préférence lorsque la bandelette urinaire **301** est disposée dans la zone d'essai **304** du dispositif d'analyse **300.** Une étape intermédiaire préalable **B3** peut être prévue lors de laquelle l'utilisateur place la bandelette urinaire **301** dans la zone d'essai **304.** Cette étape intermédiaire **B3** est rendue optionnelle lorsque la bandelette **301** est fixée sur le dispositif d'analyse **300** avant son conditionnement dans l'emballage **302.** Une étape préalable **B4,** comprenant l'ouverture de l'emballage **302,** est également prévue avant la mise en œuvre de la méthode d'analyse selon la présente invention. L'étape intermédiaire **B2** de retrait du dispositif d'absorption **202** de la bandelette urinaire **301** s'effectue après une durée prédéterminée d'application, suffisante pour faire réagir tous les réactifs indicateurs **307** de la bandelette urinaire **301.** L'étape intermédiaire **B2** de retrait du dispositif d'absorption **202** intervient par conséquent après une durée d'humectation comprise entre 10 et 120 secondes, de préférence entre 60 et 120 secondes, et plus particulièrement entre 30 et 60 secondes. La durée d'humectation est en particulier adaptée à la vitesse de réaction des réactifs indicateurs **307.** L'humectation est interrompue après une durée suffisante pour faire réagir les réactifs indicateurs **307** et suffisamment courte pour en éviter la dégradation par l'urine.
L'étape **C** de lecture du résultat de l'analyse peut inclure une étape intermédiaire **C1** de temporisation, lors de laquelle la couleur des réactifs indicateurs **307** continue de changer après le retrait du dispositif d'absorption **202** de la bandelette urinaire **301.** La durée de temporisation peut varier de 0 à 120 secondes en fonction de la vitesse de réaction des réactifs indicateurs **307.** L'étape intermédiaire **C1** est de préférence comprise entre 30 et 60 secondes. L'étape intermédiaire de temporisation **C1** peut alternativement être laissée au libre arbitre de l'utilisateur qui peut considérer que les réactifs indicateurs **307** peuvent être lus lorsque leur couleur ne varie plus. De préférence, le résultat peut être obtenu après environ 1 minute après le début de l'humectation. L'étape **C** peut en outre comprendre une étape intermédiaire **C2** de comparaison de la couleur du ou des réactifs **307** disposés sur la bandelette urinaire **301** avec une ou plusieurs échelles colorimétriques **306.** Cette étape intermédiaire de comparaison **C2** permet d'identifier un écart éventuel par rapport à la norme, matérialisée par une couleur de référence. L'étape **C** de lecture du résultat peut en plus ou alternativement comprendre une étape intermédiaire **C3** d'analyse électronique, impliquant l'utilisation d'un dispositif électronique adapté et d'un programme d'analyse adéquat. Cette étape intermédiaire **C3** comprend une reconnaissance de la couleur des réactifs indicateurs **307** par un moyen électronique, tel qu'une caméra ou un module photographique, ainsi que l'interprétation de la couleur reconnue. Le dispositif électronique **400** peut alors informer l'utilisateur d'un écart éventuel par rapport à la norme de l'un ou plusieurs des composés et paramètres analysés, ou bien d'un non-écart par rapport à la norme. L'information peut être délivrée à l'utilisateur du dispositif électronique **400** par l'intermédiaire d'un message écrit, visible sur l'interface **401** de l'application, ou bien délivré par messagerie, ou par message audio, ou par une combinaison de messages écrit et audio. Une information simplifiée, consistant en l'identification d'un écart ou d'un non-écart par rapport à la norme de l'un ou plusieurs des composés et paramètres de l'urine, peut être matérialisée par un message « OK » en cas de non-écart, ou « Non-conforme » en cas d'écart. Tout message équivalent ou code couleur prédéterminé, tel que vert en cas de non-écart et rouge en cas d'écart, peut être utilisé. Une information plus complète permettant d'identifier le ou les composés et paramètre de l'urine, ainsi que leur statut « OK » ou « Non-Conforme » peut alternativement être délivrée à l'utilisateur du dispositif électronique ou à une personne autorisée par l'utilisateur du dispositif électronique. Bien que l'une ou l'autres des étapes intermédiaires **C2** et **C3** suffise à lire le résultat de l'analyse, elles peuvent être mises en œuvre de manière concomitante, permettant ainsi une double lecture.

En cas d'écart par rapport à la partie "Norme" des échelles colorimétriques **306,** l'analyse peut être réitérée après deux heures et au moins une heure avant ou après un repas. Les réactifs indicateurs **307** sont ceux couramment utilisés pour l'analyse des composés de l'urine. Un réactif indicateur **307** de la bandelette urinaire **301** peut par exemple contenir du nitroprussiate de sodium, de manière à détecter des cétones éventuellement contenues dans l'échantillon d'urine. D'autres réactifs indicateurs usuels peuvent être utilisés.

L'absorbeur d'humidité **3** est de préférence fixé sur le dispositif **300,** ce qui évite son influence directe sur les réactifs indicateurs **307** dans l'emballage individuel **302.**

Le dispositif d'analyse **300** est prévu pour un ou plusieurs composants ou paramètres. De préférence, le dispositif d'analyse **300** est prévu pour au moins 3 composants et/ou paramètres. Par exemple, un dispositif d'analyse **300** peut être conçu pour déterminer la présence de glucose et de cétones dans l'urine, et pour en mesurer le PH. En plus ou à la place, d'autres composants tels que leucocytes, érythrocytes et albumine peuvent être étudiés. Toutes les combinaisons sont également possibles.

La présente invention propose un kit d'analyse **K** comprenant les éléments nécessaires à la mise en œuvre de la méthode décrite ci-dessus. Le kit d'analyse **K** comprend notamment une couche de diagnostic **100** et un dispositif d'analyse **300.**
La couche de diagnostic **100** comporte un matériau absorbant sur la majeure partie de sa surface d'absorption **101.** Une zone centrale **102** de sa surface d'absorption **101** est de préférence dépourvue de matériau absorbant, ou pourvue d'une quantité plus faible de matériau absorbant. La couche **100** est pourvue d'une enveloppe externe imperméable **103,** percée de un ou plusieurs orifices **104** en vis-à-vis de la zone centrale **102,** dont les propriétés absorbantes sont éventuellement amoindries par rapport au reste de la zone d'absorption **101.** La couche de diagnostic **100** comporte en outre un élément d'accumulation **200** disposé de manière amovible sur le ou les orifices **104** de l'enveloppe imperméable **103.** L'élément d'accumulation **200** comporte un moyen de fixation amovible **201,** qui permet de le maintenir en place sur la couche **100,** et un dispositif d'absorption **202** qui permet d'absorber l'urine non retenue par la zone d'absorption **101** de la couche de diagnostic **100.** Le dispositif d'absorption **202** est de préférence solidaire du moyen de fixation amovible **201.** Alternativement, le dispositif d'absorption **202** peut être séparé du moyen de fixation amovible **201.**
Le moyen de fixation amovible **201** peut par exemple prendre la forme d'une languette pourvue d'une substance adhésive sur sa face apposée à la couche de diagnostic **100.** La languette, plus large que l'orifice ou les orifices **104** de l'enveloppe imperméable **103,** peut alors être collée à la couche de diagnostic **100** et maintenir le dispositif d'absorption **202** en face de l'orifice **104.** Le dispositif d'absorption **202** peut quant à lui comporter une ou plusieurs couches d'un matériau absorbant sous forme tissée ou non tissée. Un matériau absorbant neutre vis-à-vis des composants de l'urine à étudier est de préférence utilisé, de manière à ne pas dégrader l'échantillon à analyser. Une fibre végétale ou synthétique, ou un mélange peuvent être utilisés. Le dispositif d'absorption **202** peut en outre comprendre un ou plusieurs produits hygroscopiques, pour autant qu'ils n'interagissent pas avec les composants de l'urine recherchés, ou qu'ils ne les retiennent pas au point d'en empêcher leur réaction avec les réactifs indicateurs **307.** Dans le cas où le dispositif d'absorption **202** peut être séparé du moyen de fixation amovible **201,** il peut comprendre une interface de fixation **203** qui permette de le maintenir sur la face interne du moyen de fixation amovible **201.**

Le dispositif d'analyse **300** est de préférence préconditionné dans un emballage individuel hermétique **302.** L'emballage en question peut notamment être constitué d'une matière plastique imperméable à l'air et à l'humidité, et de préférence opaque à la lumière, de manière à préserver les réactifs indicateurs **307.** L'emballage **302** peut en outre comprendre une ou plusieurs encoches de prédécoupe, pour en faciliter l'ouverture. L'emballage **302** peut également contenir une notice explicative **310.** Alternativement, la notice explicative **310** peut être apposée à l'extérieur de l'emballage **302,** ou bien incluse dans le kit d'analyse **K** de manière indépendante.

L'emballage **302** contient le dispositif d'analyse **300,** au moins une bandelette urinaire **301,** pourvue d'au moins un réactif indicateur **307,** et un absorbeur d'humidité **3.**
Le dispositif d'analyse **300** peut être constitué d'une matière cellulosique, telle que du carton ou du papier, ou bien en matière plastique. Il peut être rigide ou souple. Il comporte de préférence une zone d'analyse, délimitée par un relief **303** visant à contenir l'excès éventuel d'urine dans la zone d'analyse. La zone d'analyse comporte au moins une zone d'essai **304,** où peut être disposée une bandelette urinaire **301.** La zone d'essai coïncide au moins en partie à une zone de disposition **305** de l'accumulateur d'urine **200,** de telle sorte que la bandelette urinaire **301** soit humectée lors de l'apposition de l'accumulateur d'urine **200.** La zone d'analyse du dispositif peut en outre contenir une ou plusieurs échelles colorimétriques **306.** Alternativement, la ou les échelles colorimétriques **306** sont placées à l'extérieur de la zone d'analyse de manière à ne pas être souillée par les éventuelles traces d'urine. Le dispositif d'analyse **300** comporte autant d'échelles colorimétriques **306** que la bandelette urinaire **301** contient de réactifs indicateurs **307.** Pour faciliter la lecture du diagnostic, les échelles colorimétriques **306** sont disposées en regard des réactifs indicateurs **307** correspondants. De manière à éviter toute erreur de lecture, la bandelette urinaire **301** est de préférence fixée au préalable sur le dispositif d'analyse **300,** de telle sorte que les réactifs indicateurs **307** soient en vis-à-vis de leur échelle colorimétrique correspondante. Les échelles colorimétriques **306** peuvent être assorties d'une légende indiquant le produit ou le paramètre auquel elle est dédiée. L'échelle colorimétrique ou les échelles colorimétriques **306** comportent de préférence une couleur de référence indiquant la norme du produit ou du paramètre correspondant. La couleur de référence correspond alors à la couleur du réactif indicateur **307** de la bandelette urinaire **301** après la mise en œuvre de la présente méthode, lorsque le composé ou le paramètre mesuré ne présente pas d'écart à la norme. L'échelle ou les échelles colorimétriques **306** comportent au moins une couleur supplémentaire, différente de la couleur de référence, et indicatrice d'un écart à la norme. Elles peuvent comporter d'autres couleurs indicatrices de plus grands écarts par rapport à la norme. Les couleurs indiquant les plus grands écarts à la norme sont de préférence disposées sur les échelles colorimétriques **306** à une distance plus importante de la couleur de référence que les couleurs indicatrices de plus faibles écarts à la norme.
La couleur des réactifs **307** de la bandelette urinaire **301** obtenue après son humectation peut alors être comparée aux couleurs de l'échelle colorimétrique correspondante, de manière à identifier un éventuel écart à la norme.
Au cas où le dispositif d'analyse 300 contient plusieurs échelles colorimétriques **306,** leurs couleurs sont spécifiques des réactifs indicateurs **307** correspondants. Elles peuvent donc varier d'une échelle colorimétrique **306** à l'autre.

Le dispositif d'analyse **300** peut en outre comporter une zone de positionnement **308** d'un absorbeur d'humidité **3.** L'absorbeur d'humidité **3** est de préférence fixé au dispositif d'analyse **300** au niveau de sa zone de positionnement **308,** au moment de son conditionnement, de manière à maintenir l'atmosphère de l'emballage individuel suffisamment sec pour limiter ou éviter la dégradation des réactifs indicateurs **307.** L'absorbeur d'humidité peut contenir des éléments hygroscopiques d'origine naturelle et/ou synthétique. Ces éléments peuvent être ensachés dans un contenant perméable à l'air ou fixés sur l'absorbeur d'humidité **3.** La fixation de l'absorbeur d'humidité **3** sur le dispositif d'analyse **300** permet d'éviter son contact avec les réactifs indicateurs **307.**

Le dispositif d'analyse **300** peut comporter en plus de la zone d'analyse, une zone d'instructions. La zone d'instructions rappelle par exemple à l'utilisateur les étapes à suivre pour effectuer l'analyse de l'échantillon d'urine ou renvoyer aux indications dispensées dans une notice d'utilisation séparée. La zone d'information peut en plus ou alternativement comporter un signe de reconnaissance digitale tel qu'un QR code, qui permette à l'utilisateur d'accéder à des fichiers numériques. De tels fichiers numériques peuvent par exemple contenir une ou plusieurs vidéos ou des instructions à suivre en cas de situation anormale. La zone d'instruction peut en outre ou alternativement comporter un espace libre permettant à l'utilisateur de faire des annotations, telles que la date et l'heure du test, ou bien le nom et le prénom de la personne origine de l'échantillon.

La notice d'utilisation incluse dans le kit d'analyse **K** contient les informations nécessaires à la mise en œuvre de la présente méthode de diagnostic. Elle peut en outre comprendre des mises en garde éventuelles et/ou des consignes de sécurité ou d'hygiène. La notice d'utilisation peut également contenir les références à un programme électronique accessible à l'utilisateur. Un tel programme peut être téléchargé sur un dispositif électronique **400,** tels qu'une tablette électronique, un téléphone portable ou tout autre dispositif électronique adapté. Un tel programme peut en particulier être une application mobile permettant de faciliter le diagnostic, via une interface adéquate **401.** Le programme permettant le diagnostic de manière électronique peut notamment comporter une ou plusieurs zones de détection **402** matérialisées sur l'écran du dispositif électronique **400,** et correspondant aux réactifs indicateurs **307** de la bandelette urinaire **301.** Le module caméra du dispositif électronique **400,** ou tout autre dispositif comparable, permet de faire coïncider ces zones de détection **402** avec les réactifs indicateurs **307** de la bandelette urinaire **301** et d'en capturer l'image. La couleur des réactifs indicateurs **307** est ainsi détectée et comparée à des valeurs de références préalablement enregistrées dans le programme électronique. Le résultat du test peut alors être rendu sous la forme de message écrit et/ou audio via le dispositif électronique **400.** Le programme électronique peut en outre comprendre une fonction de suivi des résultats pour établir un historique et déceler d'éventuelles variations dans les analyses. Les résultats d'analyse peuvent être ou non enregistrés sur un serveur distant du dispositif électronique **400** utilisé pour pratiquer le test. Des messages d'alertes peuvent en outre être prévus pour inciter l'utilisateur à effectuer le test ou à le réitérer. Le programme électronique peut comprendre un seul ou plusieurs niveaux de prestation. Un premier niveau de prestation peut être par exemple défini pour des utilisateurs occasionnels et un second niveau de prestation, proposant plus de fonctionnalités, peut être destiné aux professionnels de la santé.

Dans le cas où le kit d'analyse **K** comprend une référence à un programme électronique accessible à l'utilisateur, les échelles colorimétriques **306** du dispositif d'analyse **300** peuvent être optionnelles. Dans le cas où elles sont présentes, l'utilisateur peut comparer le résultat obtenu via le dispositif électronique **400** avec le résultat obtenu par l'observation de l'échelle colorimétrique **306,** permettant ainsi un double control.

La présente invention couvre également les éléments séparés du kit d'analyse **K** décrits ci-dessus. En l'occurrence, la présente invention comprend une couche de diagnostic **100** munie d'un accumulateur d'urine amovible **200,** ainsi que la bandelette urinaire **301.** Il est à noter que la bandelette urinaire **301** utilisée dans la présente méthode peut être de longueur réduite et comprendre des réactifs indicateurs **307** sur toute sa longueur. Il n'est en effet pas nécessaire de dédier une extrémité à sa manipulation, qui soit dépourvue de réactifs indicateurs.

La méthode selon l'invention permet, grâce au screening de l'urine, d'identifier à leurs étapes initiales, d'éventuelles perturbations de l'état de santé d'une personne. Un spécialiste peut alors être contacté pour consultation et traitement. La méthode permet également le suivi simple et régulier de disfonctionnements ou de pathologies déjà identifiés. Elle peut notamment être mise à profit pour déterminer des déviations éventuelles par rapport à l'évolution de la pathologie, ou bien pour identifier les moments les plus propices à la prise de médicaments. La présente méthode peut également être utilisée en routine lors de traitements ou de tests clinique pour le suivi de leurs effets sur l'organisme.

La couche de diagnostic **100** est notamment avantageuse pour recueillir l'urine dans les meilleures conditions de confort pour la personne dont l'urine est analysée et pour la personne pratiquant le test.

La présente méthode n'exige pas de connaissances particulières et peut être mise en œuvre à domicile à tout moment par les proches du patient. Les parents peuvent ainsi facilement suivre l'état de santé de leur enfant.

Les échelles colorimétriques **306** utilisées peuvent être identiques à celles qui existent sur le marché, ce qui n'exige pas de formation spéciale du personnel sanitaire.

### Validité de la méthode

Lors d'une étude, la présente invention est reproduite dans les conditions d'un laboratoire clinique. Le but de cette étude est de déterminer la validité des résultats des analyses de l'urine obtenus par la méthode d'humectation de réactifs indicateurs par comparaison de ces résultats avec ceux obtenus à l'aide du moyen traditionnel d'immersion de bandelette urinaires dans un récipient contenant l'urine. Dans cette étude, l'urine provenant d'une même personne est analysée par les deux moyens d'humectation et d'immersion.

50 couches avec accumulateur d'urine intégré et 50 dispositifs avec bandelettes urinaires intégrées fabriqués par la compagnie DiaSys Group pour Palma Group S.A sont utilisés pour l'étude. Dans ces dispositifs sont intégrées des bandelettes urinaires comportant des réactifs indicateurs pour le glucose, les cétones et le PH. Des bandelettes urinaires analogues produites par la compagnie DiaSys Group pour Palma Group S.A sont utilisées pour réaliser l'analyse par le moyen traditionnel.

Le principe de fonctionnement et les études concernant la couche pour le prélèvement d'échantillons biologiques sont décrits d'une manière détaillée dans les documents accompagnant la demande de brevet Nº CH 00429/17.

L'étude est réalisée de la manière suivante. Dans les conditions d'un laboratoire clinique, l'urine provenant d'une même personne est divisée en deux parties égales étudiées ensuite simultanément relativement à trois composantes: glucose, cétones et PH. Les résultats obtenus par la présente méthode d'humectation correspondent totalement à ceux obtenus par la méthode usuelle d'immersion des bandelettes urinaires dans un récipient contenant l'urine, ce qui confirme la validité de la méthode proposée.

Dans le cas de l'humectation, la couleur des réactifs indicateurs change au bout d'une minute.

L'étude de la validité des données relatives à l'identification des leucocytes et de protéine par la méthode d'humectation est réalisée dans les mêmes conditions, avec des couches à accumulateurs d'urine et des bandelettes urinaires de la compagnie AllTest Biotech.

L'urine provenant d'une même personne est analysée par les méthodes d'humectation et d'immersion. La validité des résultats est confirmée par l'étude microscopique du dépôt.

Les données obtenues confirment la validité des résultats obtenus par la méthode d'humectation dans 80% des cas en comparaison avec les résultats obtenus par la méthode traditionnelle et par la microscopisation du dépôt. La détermination des causes d'erreur des résultats montre qu'elles sont liées à la structure du tissu de l'élément accumulateur de la couche qui retient les éléments insolubles dans l'urine, ce qui démontre l'efficacité de l'analyse de l'urine par la méthode d'humectation pour l'identification des leucocytes et de protéine dans l'urine.
L'erreur des résultats dépend notamment de la compacité des fibres de l'accumulateur d'urine.

## Revendications

1. Méthode d'identification d'écarts par rapport à une norme d'un ou plusieurs composés de l'urine et de paramètres physico-chimiques, la méthode comprenant :
- Une étape de séparation (A) d'un élément d'accumulation d'urine (200) de sa couche diagnostique (100),
- Une étape d'imprégnation (B) d'une bandelette urinaire indicatrice (301), comprenant un ou plusieurs réactifs indicateurs (307), par humectation via l'élément d'accumulation de l'urine (200) et
- Une étape de lecture (C) du résultat de l'analyse,
**caractérisé en ce que** la couche diagnostique (100) comprend une enveloppe externe imperméable (103), percée de un ou plusieurs orifices (104) en vis-à-vis de sa zone centrale (102), l'élément d'accumulation d'urine (200) étant disposé à l'extérieur de la couche (100) de manière amovible sur le ou les orifices (104), l'élément d'accumulation (200) comprend un moyen de fixation amovible (201) et un dispositif d'absorption (202), le dispositif d'absorption (202) comporte une interface de fixation (203) qui le maintien associé au moyen de fixation amovible (201), le dispositif d'absorption (202) étant solidaire du moyen de fixation amovible (201),
et ce que la méthode comprend les étapes intermédiaires de séparation (A1) de l'élément de fixation amovible (201), de prélèvement (A2) du dispositif d'absorption (202) solidaire du moyen de fixation amovible (201), et de séparation (A3) du dispositif d'absorption (202) de l'interface de fixation (203).

2. Méthode selon la revendication 1, **caractérisée en ce que** l'étape de séparation inclut le décollement d'un moyen de fixation amovible (201) permettant de prélever un dispositif d'absorption (202) imbibé de l'urine à analyser.

3. Méthode selon l'une des revendications 2 ou 3, **caractérisée en ce que** le moyen de fixation amovible (301) est disposé dans une zone centrale et extérieure de la couche (100), permettant ainsi d'accéder à un échantillon d'urine sans devoir ôter la couche (100).

4. La méthode selon l'une des revendications précédentes **caractérisée en ce que** la bandelette urinaire indicatrice (301) est associée à, ou disposée sur, un dispositif d'analyse (300), comprenant une zone d'essai (304), une zone comportant une ou plusieurs échelles colorimétriques (306), une zone (305) de disposition de l'accumulateur d'urine (200), une zone (308) de disposition d'un absorbeur d'humidité et une zone (303) d'extrusion en relief.

5. La méthode selon l'une des revendications précédentes, **caractérisée en ce que** l'imprégnation de la bandelette urinaire indicatrice (301) par humectation via l'élément d'accumulation de l'urine (200) est maintenue de 10 à 120 secondes.

6. Méthode selon l'une des revendications précédentes **caractérisée en ce que** l'étape de lecture des résultats comprend une temporisation de 0 à 120 secondes.

7. Méthode selon l'une des revendications précédentes, **caractérisée en ce que** la lecture du résultat inclut la comparaison de la couleur des réactifs indicateurs (307) avec la ou les échelles colorimétriques (306) correspondantes.

8. Méthode selon l'une de revendications précédentes, **caractérisée en ce que** la lecture du résultat inclut la reconnaissance de la couleur des réactifs indicateurs (307) par un dispositif électronique (400) équipée d'une interface (401).

9. Couche diagnostique (100), **caractérisée en ce qu'**elle comprend un accumulateur d'urine (200), disposé à l'extérieur de la couche (100) et en position centrale, lequel est fixé de manière amovible à la couche (100) par un moyen de fixation amovible (201), **caractérisé en ce que** la couche diagnostique (100) comprend une enveloppe externe imperméable (103), percée de un ou plusieurs orifices (104) en vis-à-vis de sa zone centrale (102), l'élément d'accumulation d'urine (200) étant disposé à l'extérieur de la couche (100) de manière amovible sur le ou les orifices (104), l'élément d'accumulation (200) comprend un moyen de fixation amovible (201) et un dispositif d'absorption (202), le dispositif d'absorption (202) comporte une interface de fixation (203) qui le maintien associé au moyen de fixation amovible (201), le dispositif d'absorption (202) étant solidaire du moyen de fixation amovible (201).

10. Kit comprenant la couche telle que décrite dans la revendication 9, et au moins une bandelette urinaire (301).

11. Kit selon la revendication 10, **caractérisé en ce qu'**il comprend en outre une notice explicative ou une référence à un programme téléchargeable sur un dispositif électronique (400), ou une combinaison des deux.

## Patentansprüche

1. Methode zur Identifizierung von Abweichungen in Bezug auf eine Norm eines oder mehrerer Bestandteile des Urins und von physikalisch-chemischen Parametern, wobei die Methode umfasst:
- einen Schritt des Trennens (A) eines Urinsammelelements (200) von dessen Diagnosewindel (100),
- einen Schritt des Imprägnierens (B) eines Urinteststreifens (301), umfassend ein oder mehrere Testreagenzien (307), durch Befeuchten über das Urinsammelelement (200) und
- einen Schritt des Ablesens (C) des Analyseergebnisses,
**dadurch gekennzeichnet, dass** die Diagnosewindel (100) eine undurchlässige äußere Hülle (103) umfasst, die von einem oder mehreren Durchbrüchen (104) gegenüber ihrer zentralen Zone (102) durchbrochen ist, wobei das Urinsammelelement (200) außerhalb der Windel (100) auf dem oder den Durchbrüchen (104) lösbar angeordnet ist, wobei das Sammelelement (200) ein lösbares Befestigungsmittel (201) und eine Absorptionsvorrichtung (202) umfasst, wobei die Absorptionsvorrichtung (202) eine Befestigungsschnittstelle (203) aufweist, die sie mit dem lösbaren Befestigungsmittel (201) verbunden hält, wobei die Absorptionsvorrichtung (202) mit dem lösbaren Befestigungsmittel (201) fest verbunden ist,
und dass die Methode die Zwischenschritte des Trennens (A1) des lösbaren Befestigungselements (201), des Entnehmens (A2) der mit dem lösbaren Befestigungsmittel (201) fest verbundenen Absorptionsvorrichtung (202) und des Trennens (A3) der Absorptionsvorrichtung (202) von der Befestigungsschnittstelle (203) umfasst.

2. Methode nach Anspruch 1, **dadurch gekennzeichnet, dass** der Schritt des Trennens das Ablösen eines lösbaren Befestigungsmittels (201) einschließt, wodurch erlaubt wird, eine mit zu analysierendem Urin getränkte Absorptionsvorrichtung (202) zu entnehmen.

3. Methode nach einem der Ansprüche 2 oder 3, **dadurch gekennzeichnet, dass** das lösbare Befestigungsmittel (301) in einer zentralen Zone und außerhalb der Windel (100) angeordnet ist, wodurch erlaubt wird, auf eine Urinprobe zuzugreifen, ohne die Windel (100) ausziehen zu müssen.

4. Methode nach einem der vorangehenden Ansprüche, **dadurch gekennzeichnet, dass** der Urinteststreifen (301) mit einer Analysevorrichtung (300) verbunden oder auf ihr angeordnet ist, umfassend eine Testzone (304), eine Zone, die eine oder mehrere Farbskalen (306) aufweist, eine Zone (305) zum Anordnen des Urinsammlers (200), eine Zone (308) zum Anordnen eines Feuchtigkeitsabsorbers und eine Reliefextrusionszone (303).

5. Die Methode nach einem der vorangehenden Ansprüche, **dadurch gekennzeichnet, dass** das Imprägnieren des Urinteststreifens (301) durch Befeuchten über das Urinsammelelement (200) 10 bis 120 Sekunden durchgeführt wird.

6. Methode nach einem der vorangehenden Ansprüche, **dadurch gekennzeichnet, dass** der Schritt des Ablesens der Ergebnisse eine Zeitverzögerung von 0 bis 120 Sekunden umfasst.

7. Methode nach einem der vorangehenden Ansprüche, **dadurch gekennzeichnet, dass** das Ablesen des Ergebnisses das Vergleichen der Farbe der Testreagenzien (307) mit der oder den entsprechenden Farbskalen (306) einschließt.

8. Methode nach einem der vorangehenden Ansprüche, **dadurch gekennzeichnet, dass** das Ablesen des Ergebnisses die Wiedererkennung der Farbe der Testreagenzien (307) durch eine elektronische Vorrichtung (400) einschließt, die mit einer Schnittstelle (401) ausgestattet ist.

9. Diagnosewindel (100), **dadurch gekennzeichnet, dass** sie einen Urinsammler (200) umfasst, der außerhalb der Windel (100) und in zentraler Position angeordnet ist, der an der Windel (100) mit Hilfe eines lösbaren Befestigungsmittels lösbar befestigt ist, **dadurch gekennzeichnet, dass** die Diagnosewindel (100) eine undurchlässige äußere Hülle (103) umfasst, die von einem oder mehreren Durchbrüchen (104) gegenüber ihrer zentralen Zone (102) durchbrochen ist, wobei das Urinsammelelement (200) außerhalb der Windel (100) auf dem oder den Durchbrüchen (104) lösbar angeordnet ist, wobei das Sammelelement (200) ein lösbares Befestigungsmittel (201) und eine Absorptionsvorrichtung (202) umfasst, wobei die Absorptionsvorrichtung (202) eine Befestigungsschnittstelle (203) aufweist, die sie mit dem lösbaren Befestigungsmittel (201) verbunden hält, wobei die Absorptionsvorrichtung (202) mit dem lösbaren Befestigungsmittel (201) fest verbunden ist.

10. Set, umfassend die Windel nach Anspruch 9 und mindestens einen Urinteststreifen (301).

11. Set nach Anspruch 10, **dadurch gekennzeichnet, dass** es ferner einen Beipackzettel oder einen Verweis auf ein auf eine elektronische Vorrichtung (400) herunterladbares Programm oder eine Kombination der beiden umfasst.

## Claims

1. Method for identifying deviations from a norm of one or more urine compounds and physicochemical parameters, the method comprising:
- A step of separating (A) a urine accumulation element (200) from its diagnostic diaper (100),
- A step of impregnating (B) a urine indicator strip (301), comprising one or more indicator reagents (307), by wetting via the urine accumulation element (200), and
- A step of reading (C) the result of the analysis,
**characterized in that** the diagnostic diaper (100) comprises a an impermeable outer envelope (103) pierced with one or more orifices (104) opposite its central area (102), the urine accumulation element (200) being disposed outside the diaper (100) removably disposed on the orifices (104), the accumulation element (200) comprises a removable fastening element (201) and an absorption device (202), the absorption device (202) comprises a fastening interface (203) which keeps it associated with the removable fastening element (201), the absorption device (202) being integral with the removable fastening element (201),
and **in that** the method comprises the intermediate steps for separating (A1) the fastening element (201), for removing (A2) the absorption device (202) integral with the fastening element (201), and for separating (A3) the absorption device (202) from the fastening interface (203).

2. The method according to claim 1, **characterized in that** the separation step includes the detachment of a removable fastening element (201) enabling the removal of an absorption device (202) soaked in the urine to be analyzed.

3. The method according to one of claims 2 or 3, **characterized in that** the removable fastening element (301) is disposed in a central and outer area of the diaper (100), thus allowing access to a sample of urine without having to remove the diaper (100).

4. The method according to one of the preceding claims, **characterized in that** the urine indicator strip (301) is associated with, or arranged on, an analysis device (300), comprising a test area (304), an area having one or more colorimetric scales (306), an area (305) for disposing the urine accumulator (200), an area (308) for providing a moisture absorber, and an extrusion area (303) in relief.

5. The method according to one of the preceding claims, **characterized in that** the impregnation of the urine indicator strip (301) by wetting via the urine accumulation element (200) is maintained for 10 to 120 seconds.

6. The method according to one of the preceding claims **characterized in that** the step of reading the results comprises a time delay of 0 to 120 seconds.

7. The method according to one of the preceding claims, **characterized in that** the reading of the result includes the comparison of the color of the indicator reagents (307) with the corresponding color scale(s) (306).

8. The method according to one of the preceding claims, **characterized in that** the reading of the result includes the recognition of the color indicator reagents (307) by an electronic device (400) equipped with an interface (401).

9. Diagnostic diaper (100), **characterized in that** it comprises a urine accumulator (200) disposed outside the diaper (100) and in a central position, which is removably attached to the diaper (100) by removable fastening means (201), **characterized in that** the diagnostic diaper (100) comprises a an impermeable outer envelope (103) pierced with one or more orifices (104) opposite its central area (102), the urine accumulation element (200) being disposed outside the diaper (100) removably disposed on the orifices (104), the accumulation element (200) comprises a removable fastening element (201) and an absorption device (202), the absorption device (202) comprises a fastening interface (203) which keeps it associated with the removable fastening element (201), the absorption device (202) being integral with the removable fastening element (201).

10. Kit comprising the diaper as described in claim 9, and at least one urine strip (301).

11. Kit according to claim 10, **characterized in that** it further comprises an explanatory note or a reference to a downloadable program on an electronic device (400), or a combination of both.
